# EUROPÄISCHE PATENTSCHRIFT

(11) **EP 3 245 502 B1**
(45) Veröffentlichungstag und Bekanntmachung des Hinweises auf die Patenterteilung: **10.04.2019**
(21) Anmeldenummer: 16700639.4
(22) Anmeldetag: 15.01.2016
(51) Int. Cl.: G01N 21/33, G01N 21/59, C02F 1/32

(54) **VERFAHREN ZUR BESTIMMUNG DES UV-TRANSMISSIONSGRADES VON WASSER**
METHOD FOR DETERMINING THE UV TRANSMITTANCE OF WATER
PROCÉDÉ PERMETTANT DE DÉTERMINER LE DEGRÉ DE TRANSMISSION DE LUMIÈRE UV DE L'EAU

(30) Priorität: 16.01.2015 DE 102015000263
(43) Veröffentlichungstag der Anmeldung: 22.11.2017
(73) Patentinhaber: Xylem IP Management S.à.r.l., 1259 Senningerberg (LU)
(72) Erfinder: KRÜGER, Friedhelm, 32657 Lemgo (DE); KANIGOWSKI, Uwe, 42553 Velbert (DE)
(74) Vertreter: Lenzing Gerber Stute
(86) Internationale Anmeldenummer: PCT/EP2016/050765
(87) Internationale Veröffentlichungsnummer: WO 2016/113390

(56) Entgegenhaltungen:
- DE-A1- 19 908 583
- JP-A- 2005 144 382
- US-A1- 2008 105 623
- US-A1- 2010 206 787

## Beschreibung

Die vorliegende Erfindung betrifft ein Verfahren zur Bestimmung des UV-Transmissionsgrades von Wasser mit den Merkmalen des Oberbegriffs des Anspruchs 1.

UV-Strahler werden seit vielen Jahrzehnten zur Desinfektion von Trinkwasser und Abwasser in Klimaanlagensümpfen und zur Desinfektion von Arbeitsbereichen in biologischen Laboratorien eingesetzt.

In Anlagen zur UV-Wasseraufbereitung werden UV-C-Strahlungsquellen in einem Quarzglashüllrohr zur Wasserbestrahlung angeordnet. Das Hüllrohr schützt die Strahlungsquelle vor Beschädigungen von außen und gewährleistet gleichzeitig eine effiziente Betriebstemperatur.

UV-Desinfektionsanlagen umfassen im Allgemeinen eine Vielzahl von UV-Strahlern, die in einem vom Wasser durchströmten Durchflussreaktor oder einem offenen Kanal angeordnet sind. Beim Durchströmen des Reaktors oder des Kanals wird das Wasser einer hinreichenden Dosis an UV-C-Strahlung ausgesetzt, damit der gewünschte Effekt erreicht wird. Um die in das Wasser abgebende Dosis zu überwachen, ist herkömmlicherweise ein UV-Sensor im Reaktor vorhanden, der orthogonal und beabstandet zu den Strahleroberflächen angeordnet ist und der ein für die in das Wasser abgebende Strahlungsleistung repräsentatives Signal empfängt. Durch diese Messung kann auch eine Veränderung der Bestrahlungsstärke erfasst werden, aber nicht deren Ursache. Die von einem UV-Sensor detektierte Bestrahlungsstärke ist durch eine Vielzahl von Faktoren beeinflusst, wie beispielweise einer Alterung des UV-Strahlers oder des UV-Sensors, einer Belagbildung auf einem der den UV-Strahler umgebenden Quarzhüllrohr und einer Änderung der Wasserqualität.

Die Wasserqualität ist dabei von besonderem Interesse, da unter anderem von ihr die ideale UV-Dosis abhängt.

Die UV-Strahlung wird durch im Wasser gelöste Stoffe geschwächt. Vor allem Eisen, Mangan, Huminsäuren und andere organische Inhaltstoffe haben einen Einfluss auf den UV-Transmissionsgrad. Zur Bestimmung der Wasserqualität muss daher der UV-Transmissionsgrad gemessen werden. Die Schwächung der UV-Strahlung durch das Wasser ist stark von der Wellenlänge abhängig. Daher wird der UV-Transmissionsgrad anhand der für die UV-Desinfektion von Wasser wirksamen Wellenlänge von 254 nm bestimmt. Zur Messung des UV-Transmissionsgrades kommt herkömmlicherweise separate Transmissionsmesstechnik zum Einsatz.

Aus der US 6,791,092 B2 ist beispielsweise ein Transmissionsmessgerät bekannt, das eine Analysekammer für den Durchlass einer Flüssigkeit aufweist, in der ein UV-Strahler und drei in verschiedenen Abständen von dem UV-Strahler angebrachte UV-Sensoren angeordnet sind. Durch eine Auswertung der Messsignale kann über die Geometrie der Anordnung auf den Transmissionsgrad der Flüssigkeit geschlossen werden.

Typische Werte des UV-Transmissionsgrades sind für Trinkwasser 85-98%/cm und für Abwasser 50-75%/cm.

Die US 2008/0105623 A1 beschreibt eine weitere bekannte Vorrichtung zur Bestimmung des UV-Transmissionsgrades von Wasser.

Es ist Aufgabe der vorliegenden Erfindung ein Verfahren zur Bestimmung des UV-Transmissionsgrads in Wasser anzugeben, das unabhängig vom Betriebs- und Alterungszustand der Strahlungsquelle erfolgt und bei dem auf eine separate Transmissionsmesstechnik verzichtet werden kann.

Diese Aufgabe wird von einem Verfahren mit den Merkmalen des Anspruchs 1 gelöst.

Danach ist ein Verfahren zur Bestimmung des UV-Transmissionsgrades von Wasser in einer von Wasser durchströmten UV-Desinfektionsanlage vorgesehen, wobei die UV-Desinfektionsanlage eine Vielzahl von Strahleranordnungen aufweist mit jeweils einer UV-Strahlungsquelle, einem die UV-Strahlungsquelle umgebenden Hüllrohr, das an einem offenen Ende eine ringförmige Stirnfläche aufweist, und mit einem UV-C-Sensor der ohne Einfluss des Wassers die aus dem Hüllrohr austretende UV-Strahlung detektiert, und mit wenigstens einem weiteren UV-Sensor, der beabstandet zu den Hüllrohren der Strahleranordnungen angeordnet ist, wobei das Verfahren folgende Verfahrensschritte aufweist: Für wenigstens eine Strahleranordnung Messen eines Maßes der aus dem Hüllrohr austretenden UV-Strahlungsleistung mittels des UV-C-Sensors; Messen eines Maßes der transmittierten Strahlungsleistung mittels des wenigstens einen weiteren von den UV-Strahlungsquellen beabstandeten UV-Sensors; Bestimmen des Transmissionsgrads des Wassers mittels dem Maß der ausgetretenen Strahlungsleistung und dem Maß der transmittierten Strahlungsleistung. Gemäß dem Verfahren kann der Transmissionsgrad des Wassers bestimmt werden, trotzdem auf eine separate Transmissionsmesstechnik verzichtet wird. Vorzugsweise wird die UV-Strahlungsquelle von dem Hüllrohr konzentrisch umgeben.

Die UV-Desinfektionsanlage kann sowohl Teil eines geschlossenen Durchflussreaktors sein oder in einem offenen Kanal angeordnet sein.

Erfindungsgemäß steht der UV-C-Sensor in optischer Verbindung mit der Stirnfläche des Hüllrohrs, so dass die sensitive Fläche des UV-C-Sensors die aus der Stirnfläche des Hüllrohrs austretende UV-Strahlung detektiert. Die aus dem Hüllrohr austretende UV-Strahlerleistung kann somit unabhängig von äußeren Einflüssen ermittelt werden. Dabei ist als Stirnfläche die im wesentlichen ringförmige Stirnseite des Hüllrohres anzusehen, nicht etwa der gesamte kreisförmige Querschnitt.

Wenn die UV-Strahlung aus der Stirnfläche austritt, dann ist diese Strahlung über die Länge des Hüllrohres in das Material des Hüllrohres eingetreten und wird an der Stirnseite ausgekoppelt. Im Gegensatz zu Verfahren, bei denen die UV-Strahlung an einem Ende innerhalb des Hüllrohres gemessen wird, und deshalb vorwiegend Strahlung aus dem Endbereich des Strahlers erfasst wird, ist die Messung an der Stirnfläche besser geeignet, den Zustand und die aktuelle Leistung des Strahlers zu erfassen.

Es ist bevorzugt, dass der zu den Hüllrohren beabstandet angeordnete wenigstens eine weitere UV-Sensor in dem strömenden Wasser angeordnet ist.

In einer Ausführungsform wird in dem letzten Verfahrensschritt "Bestimmen des Transmissionsgrads des Wassers mittels dem Maß der ausgetretenen Strahlungsleistung und dem Maß der transmittierten Strahlungsleistung" der Transmissionsgrad des Wassers mit Hilfe der Geometrie der UV-Desinfektionsanlage bestimmt.

In einer anderen Ausführungsform wird in dem letzten Verfahrensschritt der Transmissionsgrad des Wassers durch einen transmissionswert-proportionalen Zwischenwert bestimmt wird, der sich mittels des Logarithmus des Quotienten des Maßes für die ausgetretene Strahlungsleistung und des Maßes der transmittierten Strahlungsleistung berechnet.

Es kann aber stattdessen auch vorteilhaft sein, in dem letzten Verfahrensschritt den Transmissionsgrad des Wassers mit Hilfe von Umsetzungstabellen zu bestimmen.

Vorzugsweise ist der wenigstens eine weitere zu den Hüllrohren beabstandet angeordnete UV-C-Sensor orthogonal zu einer Umfangsfläche der Hüllrohre angeordnet.

Eine bevorzugte Ausführungsform der Erfindung wird nachfolgend anhand der Zeichnung näher erläutert.

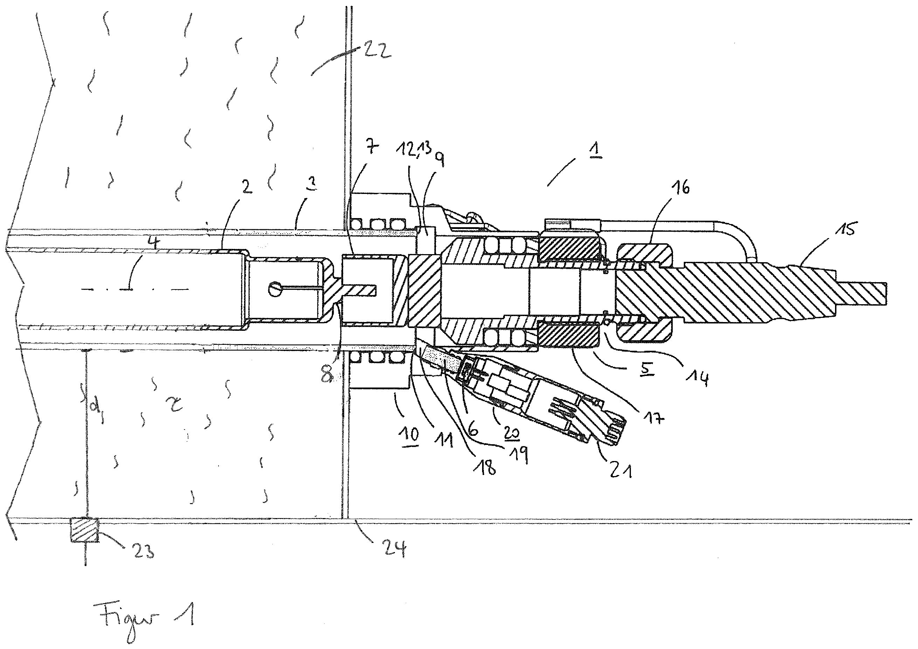

Die Figur 1 zeigt einen Längsschnitt einer Strahleranordnung 1 mit einer Strahlungsquelle 2, die von einem im wesentlichen zylindrischen Hüllrohr 3 aus UV-durchlässigem Material entlang ihrer Längsachse 4 konzentrisch umgeben ist und mit einem Verbindungselement 5 und einem UV-C-Sensor 6. Das Verbindungselement 5 weist eine Buchse 7 auf, in der ein Kontaktsockel 8 der Strahlungsquelle 2 aufgenommen ist. Die Buchse 7 durchsetzt eine kreisförmige Scheibe 9, die im Zusammenspiel mit einem das Hüllrohr 3 im Bereich der Buchse 7 umgebenden Dichtungssystem 10 als Sitz 11 für das Hüllrohr 3 an seinem offenen Ende 12 fungiert. Das Hüllrohr 3 weist eine gleichmäßige Wandstärke auf und ist an seinem Ende 12 senkrecht zu seiner Längsachse 4 abgeschnitten, wodurch sich eine ringförmige Stirnfläche 13 bildet. Die Stirnfläche 13 liegt zumindest teilweise in Anlage mit der Scheibe 9. Das Dichtungssystem 10 dichtet das Hüllrohr 3 nach außen hin ab. Die Buchse 7 des Verbindungselements 5 ist mit einem elektrischen Anschluss 14 für einen Anschlussstecker 15 der Strahleranordnung 1 verbunden. Der Anschlussstecker 15 wird mit dem Anschluss 14, der Teil des Verbindungselements 5 ist, mittels einer Überwurfmutter 16 befestigt. Zur Montage des Dichtsystems 10 ist eine zweite Überwurfmutter 17 vorgesehen.

Die Scheibe 9 weist im Bereich des Sitzes 11 für das Hüllrohr 3 eine durchsetzende Ausnehmung 18 auf, in der wenigstens zum Teil eine Einkoppeloptik 19 des UV-C-Sensors 6 angeordnet ist. Der UV-C-Sensor 6 ist über ein Verbindungselement 20 mit einem elektrischen Anschlussstecker 21 verbunden.

Das von der UV-Strahlenquelle 2 erzeugte Licht wird beim Durchtritt durch das UV-durchlässige Material des Hüllrohrs 3 gebeugt. Bevorzugt besteht das Hüllrohr 3 aus Quarzglas. Dabei kommt es teilweise zur Totalreflexion. Ein Teil des Lichts verbleibt somit im Hüllrohr 3 und wird dort hin und her reflektiert. Das Hüllrohr 3 fungiert somit als eine Art Lichtleiter. Die UV-Strahlung tritt an der Stirnfläche 13 des Hüllrohrs 3 aus und wird über einen Luftspalt mittels der Einkoppeloptik 19 dem Sensor 6 zugeführt. Es kann auch vorgesehen sein, dass die Einkoppeloptik 19 direkt an die Stirnfläche 13 ankoppelt. Weiterhin kann auch eine direkte axiale Ankopplung des Sensors 6 an die Stirnfläche 13 mit oder ohne Luftspalt sinnvoll sein. In allen Fällen ist es vorteilhaft, wenn der UV-Sensor 6 einen Kantenfilter aufweist. In der hier dargestellten Ausführungsform wird nur ein Teil des aus der ringförmigen Stirnfläche 13 austretenden Lichtes für die Messung verwendet.

In einer anderen Ausführungsform ist vorgesehen, dass geeignete optische Übergangsstücke eingesetzt werden, die eine flächendeckendere oder sogar eine vollständige Ausnutzung der von der ringförmigen Stirnseite 13 abgegebenen Strahlung ermöglichen.

Die ankoppelnde Fläche des Sensors 6 bzw. der Einkoppeloptik 19 muss nicht zwangsläufig orthogonal zur Längsachse der Strahlungsquelle 4 verlaufen.

Vorzugsweise weist der UV-C-Sensor 6 eine Siliziumkarbid (SiC) Diode auf.

Die UV-Strahleranordnung ist Teil eines Durchflussreaktors einer UV-Strahlungsanlage. Die Verbindungselemente und die elektrischen Anschlüsse der UV-Strahleranordnung ragen dabei aus dem Wasser 22 heraus. Neben dem der UV-Strahleranordnung zugeordneten UV-C-Sensor 6 ist ein weiterer UV-Sensor 23 vorgesehen, der beabstandet zum Hüllrohr 3, beispielsweise wie hier dargestellt, im Bereich der Reaktor begrenzenden Wandung 24 angeordnet ist. Dieser beabstandet angeordnete UV-Sensor 23 ist herkömmlicherweise zur Messung der Bestrahlungsintensität im Wasser vorgesehen. Er ist dabei mit seiner Messachse vorzugsweise orthogonal zur Oberfläche des Hüllrohrs 3 ausgerichtet.

Der Wassertransmissionsgrad τ einer Schichtdicke d kann mit Hilfe des Lambert-Beerschen Gesetzes durch Bestimmung der eintretenden und transmittierten Strahlungsleistung ermittelt werden. Die UV-Strahleranordnung 1 misst die Strahlungsintensität unabhängig von dem Wassertransmissionsgrad τ. Somit lässt sich die in das Wasser eintretende absolute Strahlungsleistung unter Berücksichtigung der Reaktorbauart angeben. Der beabstandet zum Hüllrohr 3 angeordnete Sensor 23 empfängt ein für die transmittierte Strahlungsleistung repräsentatives Signal. Sollte die Strahlungsquelle 2 während des Betriebes Schwankungen unterworfen sein, wirkt sich dies auf die beiden Messgrößen der beiden Sensoren 6, 23, die ein Maß für die ausgetretene Strahlungsleistung und ein Maß für die transmittierte Strahlungsleistung angeben, gleichsam aus. Der Logarithmus des Quotienten der Strahlungswerte der beiden UV-C-Sensoren 6, 23 liefert dann ein transmissionswert-proportionales Ergebnis, welches über die Geometrie der Anlage normiert werden kann.

Es kann aber auch vorgesehen sein, die Messung des Transmissionsgrades des Wassers durch eine Messung mit beispielsweise Reinstwasser zu normieren. Anhand einer Umsetzungstabelle (Englisch: Look-up-table) kann dann von dem gemessenen Maß für die ausgetretene Strahlungsleistung und dem Maß für die transmittierte Strahlungsleistung auf den Transmissionsgrad des Wasser geschlossen werden.

Nach der Erstinstallation einer UV-Desinfektionsanlage mit einer Vielzahl von erfindungsgemäßen UV-Strahleranordnungen wird eine Normierungsmessung vorgenommen, bei der das von den UV-Sensoren gemessene Signal aufgezeichnet wird. Diese Normierungsmessung erlaubt es unabhängig von den zuvor genannten Ausführungsformen, die relative Strahlungsintensität der Einzelstrahler im Betrieb der Anlage zu bestimmen. Im Betrieb einer Mehrstrahleranlage sind somit eine Messung des Wassertransmissionsgrades und ein direkter Vergleich von Strahlungsanteilen aller installierten Strahlenquellen möglich.

Die vorliegende Erfindung gibt ein Verfahren zur Bestimmung des UV-Transmissionsgrades in Wasser an, das unabhängig vom Betriebs- und Alterungszustand der Strahlungsquelle erfolgt und bei dem auf eine separate Transmissionsmesstechnik verzichtet werden kann. Das Verfahren ist sowohl für Ein- als auch Mehrstrahleranlagen nutzbar.

Der ausgekoppelte Strahlungsanteil der Strahleranordnung ist repräsentativ für die Strahlerleistung und ein Maß für den Strahlungsfluss der UV-Strahlung in das Wasser. Zwar tragen auch hier benachbarte Strahlungsquellen einen Signalanteile zum Messsignal des dem individuellen Strahler zugeordneten UV-C-Sensors 6 bei, aber dieser Anteil ist nahezu unbedeutend.

Der zum Hüllrohr beabstandet angeordnete UV-C-Sensor 23 misst die transmittierte Strahlungsleistung, so dass durch Auswertung der Sensoren zuverlässig und kostengünstig auf den Wassertransmissionsgrad bzw. die Wasserqualität geschlossen werden kann.

## Patentansprüche

1. Verfahren zur Bestimmung des UV-Transmissionsgrades von Wasser in einer von Wasser durchströmten UV-Desinfektionsanlage, wobei die UV-Desinfektionsanlage
- eine Vielzahl von Strahleranordnungen (1) mit jeweils
-- einer UV-Strahlungsquelle (2),
-- einem die UV-Strahlungsquelle (2) umgebenden Hüllrohr (3), das an einem offenen Ende (12) eine ringförmige Stirnfläche (13) aufweist, und
-- mit einem UV-C-Sensor (6) der ohne Einfluss des Wassers die aus dem Hüllrohr (3) austretende UV-Strahlung detektiert,
- und mit wenigstens einem weiteren UV-Sensor (23), der beabstandet zu den Hüllrohren (3) der Strahleranordnungen (1) angeordnet ist, wobei das Verfahren folgende Verfahrensschritte aufweist:
a) Für wenigstens eine Strahleranordnung (1) Messen eines Maßes der aus dem Hüllrohr (3) austretenden UV-Strahlungsleistung mittels des UV-C-Sensors (6), wobei der UV-C-Sensor (6) in optischer Verbindung mit der Stirnfläche (13) des Hüllrohrs (3) steht, so dass die sensitive Fläche des UV-C-Sensors (6) die aus der Stirnfläche (13) des Hüllrohrs (3) austretende UV-Strahlung detektiert;
b) Messen eines Maßes der transmittierten Strahlungsleistung mittels des wenigstens einen weiteren von den UV-Strahlungsquellen beabstandeten UV-Sensors;
c) Bestimmen des Transmissionsgrads des Wassers mittels dem Maß der ausgetretenen Strahlungsleistung und dem Maß der transmittierten Strahlungsleistung.

2. Verfahren nach Anspruch 1, **dadurch gekennzeichnet, dass** der zu den Hüllrohren (3) beabstandet angeordnete wenigstens eine weitere UV-Sensor (23) in dem strömenden Wasser angeordnet ist.

3. Verfahren nach einem der vorhergehenden Ansprüche, **dadurch gekennzeichnet, dass** in dem Verfahrensschritt c) der Transmissionsgrad des Wassers mit Hilfe der Geometrie der UV-Desinfektionsanlage bestimmt wird.

4. Verfahren nach einem der vorhergehenden Ansprüche, **dadurch gekennzeichnet, dass** in dem Verfahrensschritt c) der Transmissionsgrad des Wassers durch einen transmissionswert-proportionalen Zwischenwert bestimmt wird, der sich mittels des Logarithmus des Quotienten des Maßes für die ausgetretene Strahlungsleistung und des Maßes der transmittierten Strahlungsleistung berechnet.

5. Verfahren nach einem der vorhergehenden Ansprüche 1 oder 2, **dadurch gekennzeichnet, dass** in dem Verfahrensschritt c) der Transmissionsgrad des Wassers mit Hilfe von Umsetzungstabellen bestimmt wird.

6. Verfahren nach einem der vorhergehenden Ansprüche, **dadurch gekennzeichnet, dass** der wenigstens eine weitere UV-C-Sensor (23) orthogonal zu einer Umfangsfläche der Hüllrohre (3) angeordnet ist.

## Claims

1. Method for determining the UV transmittance of water in a water-perfused UV disinfection system, whereby the UV disinfection system comprises
- a plurality of radiator arrangements (1) with respectively
-- a UV radiation source (2),
-- a cladding tube (3) surrounding the UV radiation source (2), comprising an annular end face (13) at an open end (12), and
-- with a UV-C sensor (6) which detects the UV radiation emitted from the cladding tube (3) without the influence of the water,
- and with at least one further UV sensor (23) which is arranged spaced apart from the cladding tubes (3) of the radiator arrangements (1), whereby the method comprises the following method steps:
a) Taking a measurement of the UV radiation output emitted from the cladding tube (3) by means of the UV-C sensor (6) for at least one radiator arrangement (1), wherein the UV-C sensor (6) is in optical connection with the end face (13) of the cladding tube (3), so that the sensitive surface of the UV-C sensor (6) detects the UV radiation emitted from the end face (13) of the cladding tube (3);
b) Taking a measurement of the transmitted radiation output by means of the at least one further UV sensor spaced apart from the UV radiation sources;
c) Determining the transmittance of the water by means of the measurement of the emitted radiation output and the measurement of the transmitted radiation output.

2. Method according to claim 1, **characterised in that** the at least one further UV sensor (23), arranged spaced apart from the cladding tubes (3), is arranged in the flowing water.

3. Method according to any one of the preceding claims, **characterised in that** in the method step c) the transmittance of the water is determined with the aid of the geometry of the UV disinfection system.

4. Method according to any one of the preceding claims, **characterised in that** in the method step c) the transmittance of the water is determined by an intermediate value proportional to the transmission value, which is calculated by the logarithm of the quotient of the measurement for the emitted radiation output and of the measurement for the transmitted radiation output.

5. Method according to either one of the preceding claims 1 or 2, **characterised in that** in the method step c) the transmittance of the water is determined by means of conversion tables.

6. Method according to any one of the preceding claims, **characterised in that** the at least one further UV-C sensor (23) is arranged orthogonal to a peripheral surface of the cladding tubes (3).

## Revendications

1. Procédé permettant de déterminer le degré de transmission de lumière UV de l'eau dans une installation de désinfection par lumière UV traversée par de l'eau, où l'installation de désinfection par lumière UV comporte
- une pluralité d'ensembles d'émetteurs (1) comportant chacun
∘ une source de rayonnement UV (2),
∘ une gaine tubulaire (3) entourant la source de rayonnement UV (2) et présentant une face frontale (13) annulaire au niveau d'une extrémité ouverte (12), et
∘ un capteur de rayonnement UV-C (6) qui détecte, sans action de l'eau, le rayonnement UV provenant de la gaine tubulaire (3),
- et au moins un autre capteur de rayonnement UV (23) qui est agencé à distance des gaines tubulaires (3) des ensembles d'émetteurs (1), le procédé comportant les étapes de procédé suivantes :
a) pour au moins un ensemble d'émetteurs (1), la mesure, au moyen du capteur de rayonnement UV-C (6), de la puissance de rayonnement UV sortant de la gaine tubulaire (3), le capteur de rayonnement UV-C (6) étant en liaison optique avec la face frontale (13) de la gaine tubulaire (3), de sorte que la surface sensible du capteur de rayonnement UV-C (6) détecte le rayonnement UV sortant de la face frontale (13) de la gaine tubulaire (3) ;
b) la mesure d'une valeur de la puissance de rayonnement transmise, au moyen d'au moins un autre capteur de rayonnement UV distant des sources de rayonnement UV ;
c) la détermination du degré de transmission de l'eau au moyen d'une mesure de la puissance de rayonnement sortant et de la mesure d'une puissance de rayonnement transmise.

2. Procédé selon la revendication 1, **caractérisé en ce qu'**au moins un autre capteur de rayonnement UV (23), en plus de ceux agencés à distance des gaines tubulaires (3), est agencé dans l'eau s'écoulant.

3. Procédé selon l'une des revendications précédentes, **caractérisé en ce que** dans l'étape de procédé c) le degré de transmission de l'eau est déterminé à l'aide de la géométrie de l'installation de désinfection par lumière UV

4. Procédé selon l'une des revendications précédentes, **caractérisé en ce que** dans l'étape de procédé c) le degré de transmission de l'eau est déterminé par une valeur intermédiaire proportionnelle à la valeur de transmission qui se calcule au moyen du logarithme du quotient de la mesure pour la puissance de rayonnement sortant et de la mesure de la puissance de rayonnement transmise.

5. Procédé selon l'une des revendications 1 ou 2, **caractérisé en ce que** dans l'étape de procédé c) le degré de transmission de l'eau est déterminé à l'aide d'un tableau de transposition.

6. Procédé selon l'une des revendications précédentes, **caractérisé en ce que** le au moins un autre capteur de rayonnement UV-C (23) est agencé de manière orthogonale par rapport à une surface circonférentielle de la gaine tubulaire (3).
